# EUROPEAN PATENT APPLICATION

(11) **EP 2 474 829 A1**
(43) Date of publication of application: **11.07.2012**
(21) Application number: 11000083.3
(22) Date of filing: 07.01.2011
(51) Int. Cl.: G01N 33/543, G01N 33/68, B82Y 5/00

(54) **Method for determination of molecular structures and functions**

(71) Applicant: Axetris AG, 6056 Kägiswil (CH)
(72) Inventor: Voeroes, Janos, 8050 Zürich (CH); Di Berardino, Marco, 6026 Rain (CH); Sugihara, Kaori, 8050 Zürich (CH); Zambelli, Tomaso, 8006 Zürich (CH)
(74) Representative: Klocke, Peter

(57) **Abstract**

The present invention describes a method for measuring transport properties of cell membranes or lipid bilayers by providing at least a substrate having a topside and a backside and plurality of nano- or micro-pores and a cell membrane covering the plurality of pores being accessible from both sides of the cell membrane for measurement, a material layer at least in the region of the pores to support the cell membrane at the pores but not hindering the transport through the cell membrane and the pores arranged on the substrate, either on the topside or on the backside, applying a fluid containing at least one molecule to the topside of the membrane in order to allow the molecule to move through the membrane, one of the pores and the material layer, and monitoring of the molecules having passed the material layer by using an optical detection method. The invention allows the monitoring of long lasting passive diffusion processes or kinetic and toxicological studies on functional membrane transport proteins.

## Description

The present Invention concerns a method for measuring transport processes across cell membranes or lipid bilayers comprising at least a substrate having a topside and a backside and plurality of nano- or micro-pores and a cell membrane covering the plurality of pores being accessible from both sides of the cell membrane.

A wide variety of important biological reactions such as energy conversion, storage, immunological reactions and signal transfers take place at cell membranes and are carried out by membrane proteins. On the other hand, the permeability of drugs through the membrane itself is a critical issue in pharmaceutical industries since many drugs have to penetrate the membrane to reach their targets inside the cells. However, conventional methods to study those membrane proteins (whole cell patch-clamp methods) and membrane permeability (Caco-2 or PAMPA permeability assays) have many drawbacks, which complicate their use as standard tools for drug screening processes.

A chip can be used for monitoring membrane transport processes, especially drug permeation through biological membranes or ion transport through ion-channel-containing membranes. Such a chip is known from WO 2006/064342, which describes in detail the background for the use and benefit of that kind of chips. It also describes the membrane, which is a cell membrane or a lipid bilayer, as a biological effective layer, as well as function and structure of the chip.

An important group of membrane proteins are the G-protein coupled receptors (GPCRs), which constitute the largest subgroup of cell membrane receptors. About half of these receptors are considered to be targets for drugs. GPCRs work as signal transduction proteins that transmit signals (such as photons, odorants, hormones, and neurotransmitters) from the outside of a cell to the interior, where reaction cascades of various kinds are triggered by the corresponding G-proteins. G-proteins are anchored covalently to the lipid bilayer and are currently classified in four families according to the nature of the α-subunits, which interact with different target membrane proteins like enzymes or ion channels. After the dissociation of the G-protein the α-subunit laterally diffuses within the lipid bilayer, binds to the target protein and activates it. In almost all cases of the *in vitro* assay systems as outlined below, bio membranes of unknown composition are immobilized on solid supports, which often lead to a restricted fluidity of the lipid bilayer and a structural disturbance of the membrane protein. For that reason, these circumstances are greatly undesired in broad drug screening campaigns, because during the assay the native functions of the proteins are potentially highly disturbed.

A second important class of membrane proteins are ion channels. These either voltage- or ligand-gated channels are characterised by the fact that they direct large amounts of ions across the membrane (up to 10⁸ ions/see), down to their electrochemical gradient. This is achieved through a conformational change of these proteins, which can open and close within milliseconds their channel structure. Many known diseases, such as heart failure, are related to the dysfunction of specific ion channels, which are therefore also being fervently investigated: The method of choice for functional and structural studies on ion channels is the patch clamp technique. This technique consists of an electrochemical measurement that takes advantage of the high ion transport rate of these proteins, by placing an electrode on each side of the membrane. By the use of the proposed highly sophisticated assay systems, a deeper insight into the function of membrane proteins of interest will be achieved. Both, academic research and drug discovery will profit from this knowledge.

A third important class of membrane proteins are the transport proteins. In contrast to the ion channels, they are able to actively transport, under energy consumption, ions or other molecules against an electrochemical gradient across a biological membrane. As compared to ion channels, however, the transport speed (rate) of these proteins is much lower than that of ion channels (max. 10⁴ /sec for transporters vs. 10⁸/sec for channels), making active transporters unsuitable for electrochemical measurements.

Beside protein-mediated signal transduction and transport processes, another possibility exists for molecules to pass a lipid bilayer: simple (passive) diffusion. This is important, on the one hand, for molecules like O₂ and CO₂, which have to cross membranes at high rates, but which can do so unaided by virtue of being small and poorly solvated, and on the other hand for many drugs, for which no specific carriers exist to facilitate cell permeation. The ability of a drug to be absorbed into the blood stream by passing through the cells lining the intestines, therefore, represents a significant parameter in the drug profiling process of pharmaceutical companies. Since the majority of drugs (>80%) enter the blood stream by passive diffusion through the intestinal epithelium, assays that predict passive permeation of orally administered drugs become increasingly important.

The determination of passive diffusion of drug candidates is usually performed by measuring the concentration of the respective compounds by UV-spectrophotometric assays or by LC-MS (Liquid Chromatography-Mass Spectroscopy) analyses, if the compounds do not absorb UV-light. For the UV-spectrophotometric analysis, neither the artificial membrane assays mentioned above, nor the Caco-2 cell-based assays provide the possibility to determine concentrations online, since the impregnated filter is optically Incompatible for transmission measurements. Any other unusual known determination method also can be used, if applicable.

The prior art chip mentioned above is used for electrochemical measurements across the membrane pores. Especially the passive diffusion process needs a membrane, which is stable over the total measuring period of many hours. The known membrane lacks on sufficient stability In the region of the pores which influences the measurement results in an undesired manner. The substrate comprises at least a surface of silicon nitride.

Therefore, the object of the present invention is to provide an improved method for optical measuring in chip-based membrane systems for biosensor and drug screening applications, which is compatible to industrial production and general usage.

The problem is solved by a method as claimed in claim 1. Further advantageous embodiments are claimed in the subclaims.

According to the invention, the method comprises
providing a chip with at least a substrate having a topside and a backside and at least one nano- or micro-pore and a membrane covering the at least one pore; providing a material layer at least in the region of the at least one pore to support the membrane at the at least one pore to allow the transport through the membrane and the pores;
applying a fluid containing at least one molecule or ions to the topside of the membrane in order to allow the molecule or ions to move through the membrane, the at least one pore and the material layer; and
monitoring of the molecules or ions having passed the membrane, the at least one pore and the material layer by using an optical detection. The nano-pore substrate is potentially of silicon and carbon containing materials but also a polymer, a metal, a dielectric, a glass or a ceramic. The material layer is provided at least in the region of the at least one pore to support and stabilize the membrane at the at least one pore but not hindering the transport through the membrane and the pores. The material layer can generally be arranged on the backside or topside of the substrate. It has some advantages during the production process to provide the material layer at the backside of the substrate, however, it is also possible to provide a working chip with the material layer at the topside of the substrate. By monitoring the molecules, the concentration of the molecules in the fluid applied to the topside of the membrane and reaching the backside is detected. In general the optical detection provides information whether the candidate is able to pass the membrane and the corresponding kinetic data. The optical detection may be performed especially by a spectroscopy method, preferably by UV/VIS absorption or fluorescence spectroscopy. For the latter, the analysed molecules can either be autofluorescent or bear a specific fluorescent tag. It is also possible that the measured fluorescence signal is influenced (amplified or quenched) by the transport of molecules through the membrane. Of special interest is the time; the candidate needs to pass the membrane. Depending on the mechanism (passive diffusion, ion channel or active transport protein) the passing time varies in a broad range. The method according to the invention allows a real-time measurement for long lasting diffusion processes that might take, for example, 48 hours or more, in view of the stable material layer.

The method provides said material layer made of any kind of a porous material, which sufficiently supports the cell membrane but is porous enough to allow a substance passed through the cell membrane also to pass through the material layer. All materials with such a characteristic known to person skilled In the art, preferably such as a polymer, a gel or a porous bulk material, are appropriate to support and stabilize the membrane in the region of the pores. A polymer material layer may include non-charged polymers such as alginate, for example, or charged polymers such as polyelectrolyte multi-layers (PEM). The latter might be fabricated by a layer-by-layer method with different polyelectrolytes. For example, the polyelectrolytes may be selected from the group of polyetherimid (PEI), polyallyamin (PAH), polyglutamatacid (PGA) or polystyrolsulfonat (PSS). It is important, that PEM films promote lipid bilayer formation either by exploiting the PEM for the fusion or by exploiting the SiN surface of the chip. In the latter case, PEM should not disturb the SiN vesicle interaction. Further, PEM films have to be permeable to ions and not to disturb the channel transport measurements, and be compatible with the ion channels as well.

Preferably the polymer is a polyelectrolyte or a hydrophilic polymer, which is fabricated with a well-known layer-by-layer method; the gel is hydro-gel, and the bulk-material a micro-/nano-porous silicon or metal or ceramic,

The material layer is advantageously deposited by well-known spinning or spraying techniques or is a sintered layer.

According a preferred embodiment, the method provides two compartments separated by said nano-pore chip with said material layer, preferably wells of micro titer plate formats, either in horizontal (within one well) or vertical (between two wells), allowing in the latter case for the direct measurement of compound concentrations via UVNIS absorption or fluorescence spectroscopy and therefore also the online determination of kinetic data. A fluid with molecules moves through the pores from one compartment into the other compartment. By applying fluorescence spectroscopy with transport molecules, the fluorescence in the other compartment is influenced, either increased by initiating fluorescence or decreased by quenching the existing fluorescence in this compartment.

The invention allows the monitoring of long lasting passive diffusion processes or kinetic and toxicological studies on functional membrane transport proteins. Therefore, the method according to the invention can be used in a drug discovery process with respect of the full functionality of membrane proteins in response to potential drug compounds to be screened. The invention is applicable to all groups of membrane proteins as mentioned above.

In the following the invention is described in detail in connection with a preferred embodiment In connection with the drawings. Single features of the invention can be realized alone or in connection with other features of the description or the claims. The drawings depict schematically in
- Figure 1: a cross-sectional view of the chip provided by the method according to the invention with a material layer on a backside of a substrate,
- Figure 2: a cross-sectional view of the chip provided by the method according to the invention with a material layer on a topside of a substrate, and
- Figure 3: a schematic view of an embodiment of the invention using two compartments and UV photo-spectroscopy.

The figures show a chip 1 with a substrate 2 with a pore 3. The chip 1 comprises a plurality of such pores 3 and comprises in an embodiment according to WO 2005/064342 an array substrate of 100 mm² total area is made potentially of silicon and carbon containing materials but also a polymer, a metal, a dielectric, a glass or a ceramic. Suitable is insofar meant as a definition that the properties of the support material do allow adhesion of a membrane 4 with the substrate. A pore array section of, for example, 400 x 400 µm comprises pores having diameters in the range of 50 to 2000 nm. The distance of the pores 3 to each other (the pitch) is chosen to be in the range of their diameter. This guarantees a comparably high molecule density of membrane proteins and the compounds to be screened diminishing utterly the amount of membrane proteins and the compounds to be screened as well.

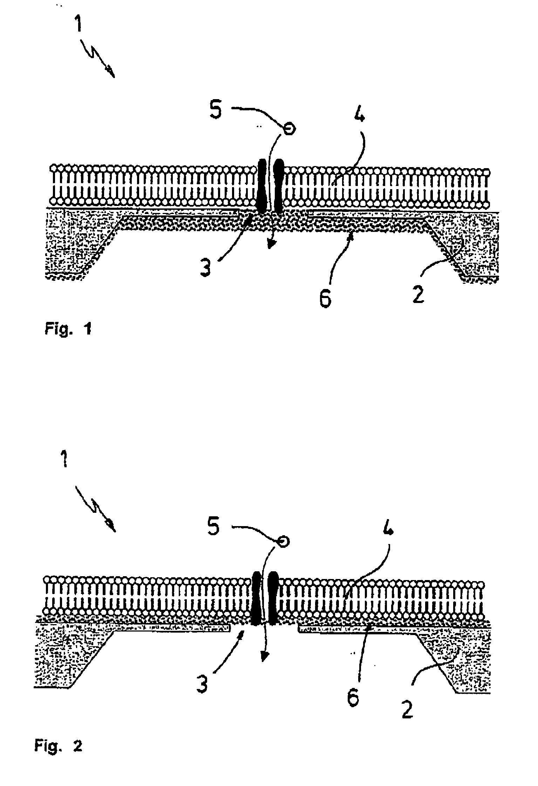

in figure 1 the membrane 4 is directly attached to the topside of substrate 2. The membrane 4 is also well-known from WO 2005/064342 and a molecule 5 passes exemplary through the membrane 4 and the pore 3. On the backside of the substrate 2 is a material layer 6 deposited by well-known deposition techniques, which covers the backside and penetrates in the pore 3 (and all other pores) in order to support and stabilize the membrane 4 in the region of the pore 3. The material layer 6 is of any kind of a porous material, which sufficiently supports the cell membrane 4 but is porous enough to allow a substance passed through the cell membrane 4 also to pass through the material layer 6. In the present case it is a PEM made of PSS/PAH. This pair promotes the vesicle fusion. However, also other couples will work.

Figure 2 shows the material layer 6 arranged between the substrate 2 and the membrane 4. In both embodiments of the figures, the material layer 6 is deposited on the substrate 2 before the membrane 4 is applied.

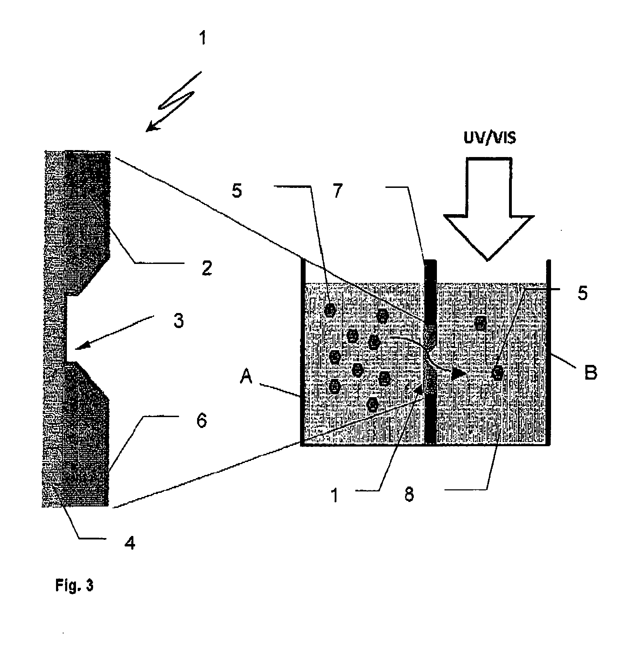

Figure 3 depicts in a schematic way a nano-pore chip 1, which separates in a wall 7 two compartments A and B, preferably wells of microtiter plate formats, either in horizontal (within one well) or vertical (between two wells) allowing in the latter case for the direct measurement of compound concentrations via UV spectrophotometry and therefore also the online determination of kinetic data. The concentration of molecules 5 in a fluid 8 in the compartment A is at the beginning higher than in the compartment B. At the end of the passive diffusion procedure in both compartments, ideally, the concentration is the same. By monitoring the kinetic data, the permeability coefficient is determined.

## Claims

1. A method for measuring transport properties of cell membranes or lipid bilayers comprising
providing a chip (1) with at least a substrate (2) having a topside and a backside and at least one nano- or micro-pore (3) and a membrane (4) covering the at least one pore (3);
providing a material layer (6) at least in the region of the at least one pore (3) to support the membrane (4) at the at least one pore (3) to allow the transport through the membrane (4) and the at least one pore (3); applying a fluid (8) containing at least one molecule (5) or ions to the topside of the membrane (4) in order to allow the molecule (5) or the ions to move through the membrane (4), at least one pore (3) and the material layer (6); and
monitoring of the molecules (5) or ions having passed the material layer (6) by using an optical detection method.

2. The method of claim 1, **characterized in that** the optical detection is performed by UVNIS absorption or fluorescence spectroscopy.

3. The method of claim 1 or 2, **characterized by** providing the material layer (6) made of any kind of a porous material, which sufficiently supports the membrane (4) but is porous enough to allow a substance passed through the membrane (4) also to pass through the material layer (6), such as a polymer, a gel or a porous bulk material.

4. The method of claim 3, **characterized in that** the polymer is a polyelectrolyte or a hydrophilic polymer.

5. The method of claim 3, **characterized in that** the gel is a hydro-gel.

6. The method of claim 3, **characterized in that** the bulk-material is a micro-/nano-porous silicon or metal or ceramic.

7. The method of anyone of the preceding claims, **characterized in that** the material layer (6) is deposited by spinning or spraying.

8. The method of anyone of the preceding claims 1 to 6, **characterized in that** the material layer (6) is produced by sintering.

9. The method of anyone of the preceding claims, **characterized by** providing two compartments (A, B) separated by the chip (1) for direct measurement of compound concentrations in one compartment (B), for applications to investigate or detect macroscopically molecular processes between said two compartments (A, B).

10. The method of anyone of the preceding claims, **characterized by** optical real-time measuring of molecules (5) or ions passing through the chip (1) for short and long lasting time periods.
